# EUROPEAN PATENT APPLICATION

(11) **EP 4 410 249 A1**
(43) Date of publication of application: **07.08.2024**
(21) Application number: 23154567.4
(22) Date of filing: 01.02.2023
(51) Int. Cl.: A61F 5/01, A61H 1/02

(54) **USER WEARABLE ORTHOSIS AND METHOD OF MANUFACTURING A USER WEARABLE ORTHOSIS**

(71) Applicant: Universität Heidelberg, 69117 Heidelberg (DE); Morgalla, Maximilian, 69214 Eppelheim (DE)
(72) Inventor: MORGALLA, Maximilian, 69214 Eppelheim (DE); ALICEA, Ryan, 69120 Heidelberg (DE); MASIA, Lorenzo, 69120 Heidelberg (DE)
(74) Representative: Müller-Boré & Partner Patentanwälte PartG mbB

(57) **Abstract**

The disclosure relates to a user wearable orthosis comprising a support structure configured to be worn on a hand of a user, wherein the support structure comprises a core skeleton layer configured to guide at least one tendon between an actuation unit and at least one end effector mounted on the hand, and at least one additional layer at least partially covering the core skeleton layer, wherein the at least one additional layer has a stiffness lower than a stiffness of the core skeleton layer. Furthermore, a method of manufacturing a user wearable orthosis is disclosed.

## Description

The invention relates to a user wearable orthosis and a method of manufacturing a user wearable orthosis.

The invention lies in the field of user wearable orthoses, and in particular in the field of tendon-driven wearable glove orthoses. Specifically, members of a population suffering from some form of, for example, upper body paralysis from a stroke, spinal cord injury, brachial plexus injury, or other causes may struggle to perform certain movements, such as grasping movements normally executed by a hand. To aid in performing such movements or restoring the ability to perform said movements, different types of mechanical devices have previously been developed, such as powered glove devices to be worn on the hand of a user. The powered glove devices may be actuated using tendon-based and pneumatic systems.

In particular, present conventional powered glove devices are typically fabric based for improved wearing comfort. However, it was recognised during the making of the present invention that such fabric-based gloves suffer from several significant drawbacks.

On one hand, any necessary hardware, such as transmission hardware, is required to be sewn onto or into the fabric, which is a delicate and time-consuming process prone to errors, particularly for sensitive hardware. Furthermore, replacing damaged hardware of a powered glove device and/or repairing damage to the fabric based powered glove device may be highly labour intensive and costly, and may even cause replacement of the entire powered glove device to become necessary.

On the other hand, fabric materials are not able to absorb compressive forces without buckling and/or folding, and thus are prone to change shape during operation of the powered glove device. While this can lead to discomfort to the user, this also limits the amount of safely transmittable force, which limits applicability of the powered glove device. This is of particular significance to users requiring high force transmission, such as spastic users that require high forces to enable movement of the fingers to overcome involuntary muscle movements resisting the movement of the powered glove device.

In addition, obtaining a perfect or even sufficient fit of the powered glove device is highly labour intensive and patient specific, such that conventional powered glove devices are manufactured using standard sizes, such as S/M/L, which consequently results in less than optimal fits to individual users. However, insufficiently well fit powered glove devices may lead to pressure concentrations on the hand, discomfort, pain, injury, and may even cause abandonment of the powered glove device by the user after prolonged wear.

Furthermore, should the powered glove device change shape during operation, this could potentially lead to forces no longer being correctly transmitted towards the desired locations, and therefore could lead to improper hand movements and/or user injury. For example, such a shape change could lead to a significant misalignment between powered glove device and the hand of the user. Furthermore, such a shape change may lead to an obstruction of the hand, such as for example to an obstruction of a grasping movement of the hand.

It is therefore an object of the present invention to provide a user wearable orthosis that overcomes one or more of the above disadvantages. In particular, it is an object of the present invention to provide a user wearable orthosis with improved operational characteristics, user adaptability, and structural stability. This object is solved by a user wearable orthosis and a method for manufacturing a user wearable orthosis having the features as recited in the independent claims.

In particular, the invention is defined by the independent claims, while preferred embodiments form the subject of the dependent claims.

An aspect of the disclosure relates to a user wearable orthosis, such as a tendon-driven orthosis and/or a glove orthosis, preferentially a tendon-driven glove orthosis. In particular, while the following description may be given in terms of tendon-driven glove orthoses for explanatory purposes, it is understood that other types of user wearable orthoses may, *mutatis mutandis,* similarly be implemented, such as for example foot orthoses and/or limb orthoses. Furthermore, while the terms "tendon-driven" and "tendon" are used for explanatory reasons herein in reference to useable force transfer elements, the present disclosure is not limited thereto. In particular, additional and/or alternative types of force transfer elements may be used, such as for example a combination of one or more tendons (such as artificial tendons), cables (such as Bowden cables), straps (such as high tensile strength fabric straps), and/or belts (such as elastomer belts).

The user wearable orthosis may be configured to be worn by a user, wherein the user wearable orthosis may be configured to be mountable at least partially on a hand of the user. Mountable at least partially on the hand of the user may be understood in this context as mountable on the hand of the user, wherein at least some components of the user wearable orthosis may be mountable on other parts of the user, such as for example on an arm, e.g., a lower arm, and/or a torso of the user, e.g., in a shoulder region of the user. As an example, an actuator connected to tendons connectable to the user wearable orthosis may be located and/or worn on a torso or hip of the user.

The user wearable orthosis further comprises a support structure configured to be worn on a hand of a user. The support structure may be configured to be worn substantially on a palm of the hand and a back of the hand. In particular, the support structure may be configured to be substantially planar when not worn by the user, and may be wrappable around the hand of the user to mount the support structure on the hand of the user. This may, on one hand, allow easy and safe storage of the support structure when not in use, and, on the other hand, simplify designing and/or manufacturing of the support structure (e.g., additional hardware may be more easily mountable on a planar support structure). The support structure may comprise at least one flex zone configured to flex to allow the support structure to be wrapped and/or bent around the hand of the user.

The support structure may be an integrally formed support structure. However, the support structure is not limited thereto. For example, the support structure may comprise one or more support sub-structures.

The support structure may comprise a hand body sub-structure configured to be worn on the palm of the hand and the back of the hand. The hand body sub-structure may be integrally formed. Alternatively, the hand body sub-structure may comprise a palm substructure arrangeable on the palm of the hand and a back sub-structure arrangeable on the back of the hand. The palm sub-structure and the back substructure may be fixable to one another to allow the hand body sub-structure to be worn on the hand.

The support structure may further comprise one or more finger sub-structures. Each finger sub-structure may be configured to be worn on at least one finger of the user, such as an index finger, a middle finger, a ring finger, and/or a little finger. The one or more finger sub-structures may be integrally formed with the hand body sub-structure and/or a back sub-structure. This may allow a very secure connection between the respective finger sub-structure(s) and the hand body sub-structure. Furthermore, a fit of the connection may be determined during and/or before manufacture of the support structure, and may therefore no longer need to be checked during mounting thereof on the user. Alternatively, at least one finger sub-structure may be, preferentially releasably, connectable with and/or fixable to the hand body sub-structure and/or the back sub-structure. In particular, this may allow a significantly facilitated mounting of the support structure on the user, such as for users suffering from hand spasticity. Furthermore, the support structure may be adapted to specific requirements of the user during the mounting thereof on the user. Specifically, this may allow improved use flexibility, wherein a given support structure may easily be adaptably to multiple different users.

The one or more finger sub-structures, preferably each finger sub-structure may be configured to be worn at least on a distal section, e.g., on a distal phalanx section, of the respective at least one finger. For example, most of the spastic forces in the hand may lie in one or more distal and/or middle joints of the fingers with comparatively low resistance in the proximal (metacarpal) joints. Therefore, when the at least one end effector, as further described below, may comprise one or more finger sub-structures, configuring said one or more finger sub-structures to be worn on the at least distal section may allow a more effective response to spastic resistance.

The support structure may comprise a thumb sub-structure. The thumb sub-structure may be configured to be worn on a thumb of the user. The thumb sub-structure may be integrally formed with the hand body sub-structure and/or the back sub-structure. Alternatively, the thumb sub-structure may be connectable with and/or fixable to the hand body sub-structure and/or the back sub-structure. The thumb sub-structure may be similarly configured to the finger sub-structures described herein.

The thumb sub-structure may be configured to be worn at least on a distal section, e.g., on a distal phalanx section, of the thumb of the user. Thereby, a more effective response to spastic resistance in the thumb of the user may be enabled.

At least one, preferably each, finger sub-structure, hand body sub-structure, palm substructure, back sub-structure, and/or the thumb sub-structure may be dimensioned according to the specific requirements of the user, such as for example to provide a good fit to the hand of the user. Furthermore, at least one, preferably each, finger substructure, hand body sub-structure, palm sub-structure, back sub-structure, and/or the thumb sub-structure may be formed separate from one another and/or separable from one another. In particular, it may therefore be possible to significantly improve an ease of use of the user wearable orthosis, such as for example during mounting and/or dismounting of the user wearable orthosis onto/from the hand of the user. In addition, a replaceability of at least one, preferably each, finger sub-structure, hand body substructure, palm sub-structure, back sub-structure, and/or the thumb sub-structure may therefore be also improved.

In particular, at least one finger sub-structure and/or the thumb sub-structure may be integrally formed with the hand body sub-structure, while at least one other of the one or more other finger sub-structures and/or the thumb sub-structure may be connectable with and/or fixable to the hand body sub-structure. For example, a finger sub-structure configured to be worn on a little finger of the user may be integrally formed with the hand body sub-structure, while other finger sub-structures configured to be worn on the other fingers of the hand of the user (e.g., the index finger, middle finger, and/or ring finger) and the thumb sub-structure may be releasably connectable with and/or releasably fixable to the hand body sub-structure. In particular, mounting the user wearable orthosis on the user may thereby be facilitated, while still allowing a high degree of adaptability.

However, the present disclosure is not limited to such a configuration, and other configurations may be possible. For example, a thumb sub-structure may be integrally formed with the hand body sub-structure, while one or more finger sub-structures configured to be worn on one or more fingers of the hand of the user may be releasably connectable with and/or releasably fixable to the hand body sub-structure.

The support structure may be configured as a multi-layer support structure comprising at least two layers.

The support structure further comprises a core skeleton layer configured to guide at least one tendon between an actuation unit and at least one end effector mounted on the hand. Guiding at least one tendon may be understood as at least partially restricting at least one degree of freedom of movement of the at least one tendon with respect to the core skeleton layer. For example, guiding at least one tendon may be understood as defining at least one tendon routing path (preferentially one tendon routing path for each tendon) along the core skeleton layer, wherein the at least one tendon may be moveable along the at least one tendon routing path while being restricted by the core skeleton layer in movement lateral to said at least one tendon routing path.

The core skeleton layer may have a core stiffness. Stiffness may be understood, within the scope of the disclosure unless individually differently specified, as the extent to which an object resists deformation in response to an applied force. The core stiffness may for example be an average stiffness of the core skeleton layer. The core stiffness may for example be a stiffness of the at least one tendon routing path.

The core skeleton layer may be an integral core skeleton layer, or the core skeleton layer may comprise one or more core skeleton sub-layers. In particular, one or more support sub-structures may each comprise at least one core skeleton sub-layer. For example, the hand body sub-structure may comprise a first core skeleton sub-layer and/or at least one finger sub-structure may comprise at least a second core skeleton sub-layer and/or the thumb sub-structure may comprise at least a third core skeleton sub-layer. While features and properties of the core skeleton layer are described and/or shown herein, it is understood that each core skeleton sub-layer may comprise any combination of features described and/or shown herein for the core skeleton layer.

The support structure further comprises at least one additional layer at least partially covering the core skeleton layer. In particular, the at least one additional layer may be arrangeable and/or fixable adjacent to the core skeleton layer. At least one additional layer may be arrangeable and/or fixable directly adjacent to, preferentially in touching contact to, the core skeleton layer. Additionally and/or alternatively, at least one additional layer may be arrangeable and/or fixable indirectly adjacent to the core skeleton layer, wherein one or more intermediate elements and/or components may be arrangeable and/or fixable between the respective at least one additional layer and the core skeleton layer. Non-restricting examples of the one or more intermediate elements and/or components may comprise one or more other additional layers, one or more sensors, and/or one or more tendons.

One or more additional layers may be integral additional layers, and/or one or more additional layers may comprise one or more additional sub-layers. In particular, one or more support sub-structures may each comprise at least one additional sub-layer. Additionally or alternatively, at least one additional sub-layer may be configured to cover one or more core skeleton sub-layers. While features and properties of the at least one additional layer are described and/or shown herein, it is understood that each additional sub-layer may comprise any combination of features described and/or shown herein for the at least one additional layer.

The at least one additional layer has a stiffness lower than a stiffness of the core skeleton layer. Each additional layer may have a corresponding additional layer stiffness. In other words, at least one additional layer stiffness, preferentially every additional layer stiffness, may be lower than the core stiffness.

The support structure may further comprise a first locking section and a second locking section engageable with the first locking section, wherein the support structure may be configured to be wrapped substantially around the hand of the user to bring the first locking section and the second locking section into engagement with each other to mount the support structure on the user. Wrapping substantially around the hand of the user may be understood such that at least part of the support structure may be deformed around the hand of the user, preferentially to at least partially conform to a shape of the hand.

The first locking section and the second locking section may comprise at least one hook and at least one loop configured to be fixable to one another to bring the first and second locking sections into engagement with one another. However, the first and second locking sections are not limited thereto, and other types of locking mechanisms may be implemented, such as for example Velcro-based locking mechanisms.

The first locking section may comprise at least one first core locking section provided on and/or in the core skeleton layer and the second locking section may comprise at least one second core locking section provided on and/or in the core skeleton layer. The at least one first core locking section may be configured to be engageable with the at least one second core locking section to mount the support structure and/or the core skeleton layer on the user.

The first locking section may comprise at least one first additional locking section provided on and/or in one or more of the at least one additional layer, and the second locking section may comprise at least one second additional locking section provided on and/or in the respective one or more of the at least one additional layer. The at least one first additional locking section may be configured to be engageable with the at least one second additional locking section to mount the support structure and/or the corresponding one or more of the at least one additional layer on the user.

The core skeleton layer may be formed of at least one metal (e.g., low carbon steel), at least one elastomer, at least one polymer (e.g., silicone, thermoplastic urethane (TPU), thermoplastic elastomer (TPE), and/or PA-6 (polyamide 6)), and/or at least one non-textile material. However, it is understood that the core skeleton layer is not restricted to such exemplary materials. For example, the core skeleton layer may be formed from a first base material, such as for example 85A TPU, and a second base material, such as 95A TPU, having a greater stiffness than the first base material.

At least one additional layer may be formed of at least one elastomer, at least one polymer (e.g., silicone, thermoplastic urethane (TPU), and/or thermoplastic elastomer (TPE)), at least one leather, and/or at least one textile material (e.g., a fabric such as a woven and/or knitted fabric). At least one additional layer may be formed of a low-density lattice, wherein the low-density lattice may be a foam, such as a TPU foam and/or a TPE foam. Furthermore, a first additional layer may be formed of a first material, while at least a second additional layer may be formed of a second material different from the first material. In particular, the support structure may comprise at least two additional layers formed from different materials. However, it is understood that the at least one additional layer is not restricted to such exemplary materials.

Specifically, it may therefore be possible to adapt the support structure very precisely to the needs of the user by selection of appropriate materials for the core skeleton layer and/or the at least one additional layer. Specifically, it may therefore be possible to combine one or more materials of the core skeleton layer and/or the at least one additional layer into a three-dimensional composite structure to enable very precise control over the, e.g., mechanical, properties of the user wearable orthosis. In particular, it becomes possible to increase stiffness and/or rigidity in areas affected by high loads during operation of the user wearable orthosis, while maintaining softness against the user, e.g., a skin of the user, and flexibility in sensitive areas. In addition, this allows the user wearable orthosis to withstand significantly higher loads with the same degree of softness.

The at least one additional layer may comprise at least one inner additional layer. The at least one inner additional layer may be arrangeable at least partially on and/or fixable (e.g., directly or indirectly adjacent) to an inner, first side of the core skeleton layer. In particular, the inner side of the core skeleton layer may be a user facing and/or hand facing side of the core skeleton layer, preferentially in a use state of the user wearable orthosis. In particular, the use state of the user wearable orthosis may be a state of the user wearable orthosis in which the user wearable orthosis is mounted on the user, e.g., mounted on a hand of the user. In other words, the at least one inner additional layer may be at least partially arrangeable between the core skeleton layer and the hand of the user, preferentially in the use state.

The at least one inner additional layer may comprise an inner fabric layer, wherein the inner fabric layer may be configured to prevent and/or reduce injury and/or discomfort of the user, such as for example caused by edges of the core skeleton layer during flexion of the hand. However, the inner fabric layer is not limited thereto. For example, the inner fabric layer may be configured to thermally insulate the hand of the user, e.g., to keep the hand of the user warm during low environmental temperatures, and/or the inner fabric layer may be configured to allow perspiration of the user to diffuse to the outside environment around the user (e.g., the inner fabric layer may be a high breathability fabric layer).

The at least one inner additional layer may be configured to be detachable and/or removable from the core skeleton layer. In particular, the at least one inner additional layer may therefore be easily replaceable and/or cleanable. For example, it may therefore be possible to easily detach an inner fabric layer, to wash and/or clean said inner fabric layer, and to subsequently reattach said inner fabric layer to the core skeleton layer. Therefore, such inner additional layers may improve hygienic properties and/or a lifetime of the user wearable orthosis.

The at least one additional layer may comprise at least one outer additional layer. The at least one outer additional layer may be arrangeable at least partially on and/or fixable (e.g., directly or indirectly adjacent) to an outer, second side of the core skeleton layer. In particular, the outer side of the core skeleton layer may be a side of the core skeleton layer facing away from the user and/or the hand, preferentially in the use state. In other words, the at least one outer additional layer may be at least partially arrangeable such that the core skeleton layer is arrangeable and/or positionable between the hand of the user and the at least one outer additional layer, preferentially in the use state.

The at least one outer additional layer may comprise an outer fabric layer, wherein the outer fabric layer may be configured to prevent injury and/or discomfort of the user, such as for example caused by edges of the core skeleton layer during flexion of the hand. However, the outer fabric layer is not limited thereto. For example, the outer fabric layer may be configured to thermally insulate the hand of the user, e.g., to keep the hand of the user warm during low environmental temperatures, and/or to improve a breathability of the user wearable orthosis. Furthermore, the outer fabric layer may be configured to prevent and/or reduce damage to the user wearable orthosis and/or the core skeleton layer during use of the user wearable orthosis.

The at least one outer additional layer may be configured to be detachable and/or removable from the core skeleton layer. In particular, the at least one outer additional layer may therefore be easily replaceable and/or cleanable. For example, it may therefore be possible to easily detach an outer fabric layer, to subsequently wash and/or clean said outer fabric layer, and to subsequently reattach said outer fabric layer to the core skeleton layer. Therefore, such outer additional layers may improve hygienic properties and/or a lifetime of the user wearable orthosis.

In particular, the support structure may comprise at least one inner additional layer and at least one outer additional layer, wherein the at least one inner additional layer and the at least one outer additional layer may be arrangeable such that the core skeleton layer is positioned at least partially between the at least one inner additional layer and the at least one outer additional layer.

Furthermore, the at least one inner additional layer and/or the at least one outer additional layer may be user customizable. For example, a material choice of the at least one inner additional layer and/or the at least one outer additional layer may be made in light of the individual requirements of the user, such as in relation to allergies, skin conditions, and/or physiological properties of the user, e.g., sweating and/or temperature perception. Furthermore, optical and aesthetic properties of the at least one inner additional layer and/or the at least one outer additional layer may be chosen in light of user preferences and requirements, such as to facilitate mounting of the user wearable orthosis on the user.

The at least one additional layer may further comprise at least one surrounding additional layer. The at least one surrounding additional layer may be configured to be directly fixable to the core skeleton layer and/or another one of the surrounding additional layers. The at least one surrounding additional layer may be configured to enclose the core skeleton layer at least partially. The at least one surrounding additional layer may be configured to cover at least part of the inner side and the outer side of the core skeleton layer. For example, the core skeleton layer may be at least partially embedded in the at least one surrounding additional layer.

The core skeleton layer may be manufactured and/or manufacturable using molding or additive manufacturing. For example, the core skeleton layer may be manufactured, e.g., using one or more materials, by 3D printing the core skeleton layer. Specifically, the core skeleton layer may therefore be easily and efficiently manufacturable. Furthermore, by providing a support structure having at least the core skeleton layer that is manufacturable by, e.g., 3D printing, the user wearable orthosis and/or the support structure can easily and locally be manufactured and/or adapted to the user, thereby increasing an adaptability and individualization of the user wearable orthosis.

Specifically, by allowing the core skeleton layer to be manufactured via, for example, 3D printing, the core skeleton layer may be precisely adapted to the specific requirements of the user, which may lead to a significant improvement of the fit of the user wearable orthosis. In addition, the core skeleton layer may easily be replaced without necessitating a replacement of the full user wearable orthosis.

The 3D printing of the core skeleton layer may comprise at least 3D printing a first part of the core skeleton layer using a first material and subsequently printing, on the first part, a second part of the core skeleton layer using a second material. It is appreciated that 3D printing is not limited to a first part and a second part. Additionally or alternatively, more and/or other parts may be printed. For example, a third part may be printed on the first part and/or the second part using the first material, the second material, or a third material. The parts of the core skeleton layer may for example correspond to one or more sections and/or components of the core skeleton layer. In particular, it may therefore be possible to efficiently manufacture the core skeleton layer using one, two, or more materials.

Furthermore, the present disclosure is not limited to only manufacturing the core skeleton layer using molding or additive manufacturing. Indeed, the at least one additional layer may be manufactured and/or manufacturable using molding or additive manufacturing. For example, the at least one additional layer may be manufactured, e.g., using one or more materials, by 3D printing the at least one additional layer. The 3D printing of the at least one additional layer may comprise at least 3D printing a first part of at least one additional layer using a first material and subsequently printing, on the first part, a second part of the at least one additional layer using a second material.

For example, the core skeleton layer and at least one additional layer may be both manufactured using molding or additive manufacturing, preferentially substantially simultaneously and/or substantially concurrently. For example, the core skeleton layer and at least one additional layer may be 3D printed together, wherein the at least one additional layer may, for example, comprise at least one surrounding additional layer configured to enclose the core skeleton layer at least partially.

The core skeleton layer may further comprise at least one tendon guide element. The at least one tendon guide element may be configured to guide at least one tendon along the core skeleton layer. The at least one tendon guide element may, for example, be made from a material different from a material of the core skeleton layer. For example, the at least one tendon guide element may be made from Teflon. The at least one tendon guide element may have an at least partially hoop-like and/or tube-like shape, wherein the at least one tendon may be guided within a central cavity or opening of the at least one tendon guide element. The at least one tendon guide element may be 3D printable onto and/or integrally formed with the core skeleton layer. One or more of the at least one tendon guide elements may define at least one tendon routing path. The at least one tendon guide element may define at least one tendon routing path for at least one, preferentially each, finger and/or thumb of the hand of the user.

The core skeleton layer may comprise one or more hardware mounting elements. The one or more hardware mounting elements may be configured such that one or more external hardware may be mountable to the core skeleton layer. The one or more hardware mounting elements may be configured to be engageable with the one or more external hardware. Non-limiting examples of external hardware include one or more sensors and/or electronic equipment. The one or more sensors may be configured to sense a bending angle of one or more fingers of the hand, a pressure on the palm of the hand and fingers, and/or a motion of the hand. The electronic equipment may comprise a PCB, e.g., a flexible PCB, and/or a control unit, wherein the control unit may for example be configured to control the one or more sensors and/or the actuator. The control unit may be configured as a PCB, for example a flexible PCB. The one or more hardware mounting elements may for example comprise one or more sleeve elements, hook elements, loop elements, and/or clip-on elements.

In particular, by enabling such mounting of external hardware directly on and/or in the core skeleton layer, a bulk and thickness of the user wearable orthosis may be reduced. In particular, the user wearable orthosis may comprise the one or more external hardware.

Additionally and/or alternatively, one or more sensors and/or electronic equipment may be integratable into the core skeleton layer to, for example, hide sensation of the one or more sensors and/or electronic equipment by the user and/or improve a mounting stability of the one or more sensors and/or electronic equipment. For example, one or more sensors and/or electronic equipment may be at least partially embedded into the core skeleton layer, preferentially fully embedded into the core skeleton layer. Thereby, a high structural stability of the user wearable orthosis may be achieved, and/or loss and/or unintentional removal of the one or more sensors and/or electronic equipment may be avoided.

The core skeleton layer may comprise at least one main layer section having a first stiffness. The at least one main layer section may be a physical section of the core skeleton layer.

The core skeleton layer may further comprise at least one reinforcement section having at least a second stiffness greater than the first stiffness. The at least one reinforcement section may be a physical section of the core skeleton layer different from the at least one main layer section. The at least one reinforcement section may comprise at least one section having a larger thickness than a thickness of the at least one main layer section. The at least one reinforcement section may comprise more material and/or denser material than the at least one main layer section. The at least one reinforcement section may comprise material having a greater stiffness than material of the at least one main layer section. For example, one or more reinforcement sections may comprise a reinforcement element embedded into the core skeleton layer, such as for example a low-carbon steel insert element.

The core skeleton layer may comprise at least two reinforcement sections, wherein each of the at least two reinforcement sections may have a substantially identical stiffness. Substantially may be understood in the context of the present disclosure as including variations due to, for example, environmental and/or production factors. Alternatively, at least two reinforcement sections may have different stiffnesses from one another.

The core skeleton layer may further comprise at least one flexion section having at least a third stiffness smaller than the first stiffness. The at least one flexion section may be a physical section of the core skeleton layer different from the at least one main layer section and/or the at least one reinforcement section. The at least one flexion section may comprise at least one section having a smaller thickness than the thickness of the at least one main layer section. The at least one flexion section may comprise less material and/or material of lower density than the at least one main layer section. For example, the at least one flexion section may comprise one or more through-holes through the core skeleton layer. The at least one flexion section may comprise material having a lower stiffness than material of the at least one main layer section. For example, one or more flexion sections may comprise a flexion element embedded into the core skeleton layer, such as for example a bending element and/or a hinge element.

The core skeleton layer may comprise at least two flexion sections, wherein each of the at least two flexion sections may have a substantially identical stiffness. Alternatively, at least two flexion sections may have different stiffnesses from one another.

Furthermore, a stiffness of the at least one additional layer may be lower than the first stiffness and/or the second stiffness. The stiffness of the at least one additional layer may be equal to or lower than the third stiffness, wherein the third stiffness may have a finite and/or non-trivial value. Alternatively, the stiffness of the at least one additional layer may be greater than the third stiffness.

The user wearable orthosis may further comprise the at least one end effector. Alternatively or additionally, the user wearable orthosis may be the at least one end effector. The at least one end effector may be configured to be worn on one or more of a thumb, an index finger, a middle finger, a ring finger, and a little finger of the hand of the user. The at least one end effector may in particular comprise at least one of the one or more finger sub-structures and/or the thumb sub-structure.

The at least one end effector may be fixable to the support structure. For example, the support structure and/or the core skeleton layer may comprise at least one mounting section, wherein the at least one end effector may be fixable, preferentially releasably fixable, to the at least one mounting section. As an example, the support structure and/or the core skeleton layer may comprise a thumb end effector mounting section, wherein a thumb end effector may be releasably fixable to the thumb end effector mounting section.

The user wearable orthosis may further comprise the at least one tendon. The at least one tendon may be connectable to the at least one end effector. The user wearable orthosis may further comprise the actuator, wherein the actuator is configured to exert a pulling force on the at least one tendon. In particular, by exerting the pulling force on the at least one tendon, the user wearable orthosis may be configured to assist a movement, such as a grasping movement, of the hand of the user. Furthermore, as previously discussed, the user wearable orthosis is not restricted to comprising only at least one tendon as a force transfer element, but additional and/or different force transfer elements may be provided and/or comprised.

An aspect of the disclosure relates to a method of manufacturing a user wearable orthosis comprising a support structure configured to be worn on a hand of a user. The method comprises manufacturing a core skeleton layer of the support structure configured to guide at least one tendon between an actuation unit and at least one end effector mounted on the hand. The method further comprises at least partially covering the core skeleton layer with at least one additional layer. The at least one additional layer has a stiffness lower than a stiffness of the core skeleton layer. For example, a simple and efficient manufacture of the user wearable orthosis may thereby be achieved.

The method may in particular comprise any combination of features of the user wearable orthosis, as described herein and/or shown in the appended Figures.

The method may further comprise wrapping the support structure substantially around the hand to bring a first locking section of the support structure into engagement with a second locking section of the support structure to mount the support structure on the user. Thereby, a simple and efficient mounting of the user wearable orthosis on the user may be achieved.

The step of manufacturing the core skeleton layer may further comprise manufacturing the core skeleton layer using molding and/or additive manufacturing. Thereby an easy and adaptable manufacture of the core skeleton layer may be achieved. Furthermore, by using, for example, additive manufacturing, such as 3D printing, the core skeleton layer can be locally and rapidly be produced.

The step of at least partially covering the core skeleton layer with the at least one additional layer may comprise a manufacturing of the at least one additional layer. The step of manufacturing the at least one additional layer may further comprise manufacturing the at least one additional layer using molding and/or additive manufacturing, such as for example 3D printing.

The steps of manufacturing the core skeleton layer and manufacturing of at least one additional layer may be undertaken at least partially substantially simultaneously and/or concurrently. For example, the core skeleton layer and at least one surrounding additional layer, configured to enclose the core skeleton layer at least partially, may therefore be efficiently and rapidly manufactured.

The step of manufacturing the core skeleton layer may further comprise providing at least one tendon guide element on and/or in the core skeleton layer, wherein the at least one tendon guide element may be configured to guide the at least one tendon along the core skeleton layer.

The step of manufacturing the core skeleton layer may further comprise forming at least one main layer section having a first stiffness. The step of manufacturing the core skeleton layer may further comprise forming at least one reinforcement section having at least a second stiffness greater than the first stiffness. The step of manufacturing the core skeleton layer may further comprise forming at least one flexion section having at least a third stiffness smaller than the first stiffness.

The invention will now be further explained using exemplary embodiments illustrated in the appended Figures. However, the embodiments shown in the appended Figures and/or described below are to be understood as exemplary only, and the invention is therefore not to be interpreted as limited to such exemplary embodiments.

The Figures show:
- **Figure 1:**: a schematic view of an exemplary support structure;
- **Figure 2:**: a schematic view of an exemplary support structure according to Figure 1;
- **Figure 3:**: a further exemplary core skeleton layer;
- **Figure 4A:**: a schematic view of an exemplary support structure;
- **Figure 4B:**: a horizontal cross-sectional view of the exemplary support structure according to Figure 4A;
- **Figure 4C:**: a vertical cross-sectional view of the exemplary support structure according to Figure 4A; and
- **Figure 5:**: an exemplary flow chart of a method of manufacturing a user wearable orthosis.

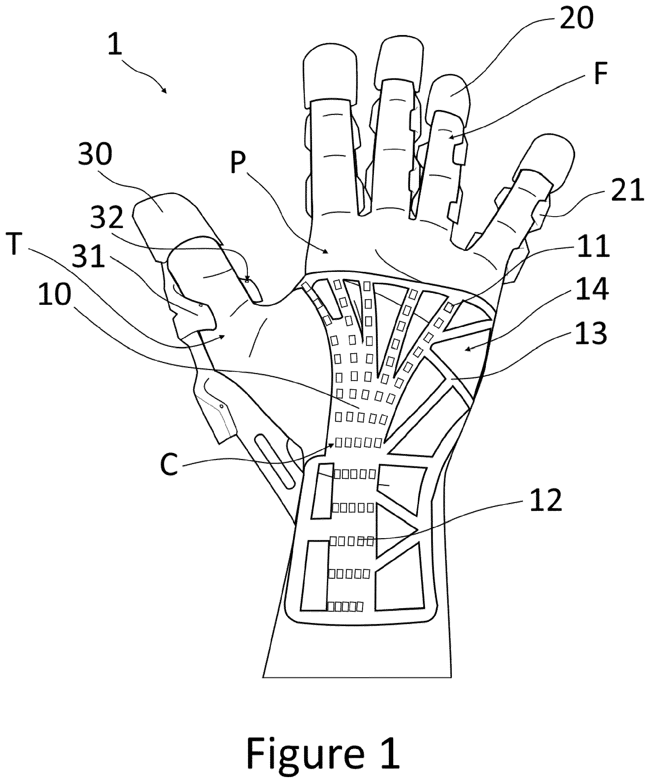

**Figure 1** shows a schematic view of an exemplary support structure **1,** wherein the support structure **1** is comprised by an exemplary user wearable orthosis **100** (shown in later Figures). In particular, the user wearable orthosis **100** is an exemplary tendon-driven glove orthosis **100** configured to be worn on a hand of a user.

The hand of the user is schematically shown in Figure 1 and comprises a palm **P** of the hand and a back (not shown) of the hand, as well as a thumb **T** and four fingers **F.**

The exemplary support structure **1** is configured to be worn on the hand of the user, and may comprise one or more support sub-structures.

The illustrated exemplary support structure **1** comprises at least a hand body substructure **10,** wherein the hand body sub-structure **10** is configured to be worn on the palm **P** and the back of the hand. In the shown example, the hand body sub-structure **10** is integrally formed and may be wrapped around the palm **P** and the back of the hand to mount the hand body sub-structure **10** on the hand.

The support structure **1** and/or the hand body sub-structure **10** further comprises a core skeleton layer **C,** in particular an integrally formed core skeleton layer **C,** configured to guide at least one tendon (not shown) between an actuation unit (not shown) and at least one end effector mounted on the hand. In the present example, the finger sub-structures **20** and the thumb sub-structure **30,** as further discussed below, may each form an end effector.

The core skeleton layer **C** may be configured to be wrappable around the hand of the user to mount the hand body sub-structure **10** and/or the core skeleton layer **C** on the hand of the user. In particular, although not explicitly shown in Figure 1 for illustrative purposes, the core skeleton layer **C** may comprise at least one first core locking section provided on and/or in the core skeleton layer **C** and at least one second core locking section provided on and/or in the core skeleton layer **C.** The at least one first core locking section may be configured to be engageable with the at least one second core locking section to mount the hand body sub-structure **10** and/or the core skeleton layer **C** on the user.

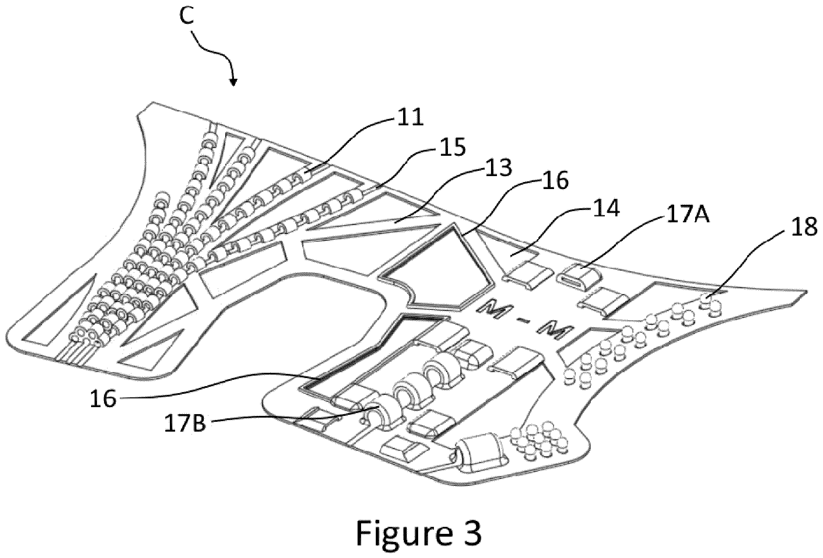

The core skeleton layer **C** may further comprise at least one tendon guide element **11.** Exemplary, schematic representations of such tendon guide elements **11** are shown in Figure 1, although it is understood that more or less of such tendon guide elements **11,** having similar and/or different shapes, may be provided (see, e.g., Figure 3). The at least one tendon guide element **11** may be configured to guide at least one tendon along the core skeleton layer **C.** The shown exemplary at least one tendon guide element **11** may be made from Teflon, wherein each tendon guide element **11** may have an at least partially hoop-like and/or tube-like shape. For example, the at least one tendon guide element **11** may be 3D printed onto and/or integrally formed with the core skeleton layer **C.** The at least one tendon guide element **11** may define at least one tendon routing path, such as the five exemplary tendon routing paths shown in Figure 3 and further described below, wherein each tendon guide element **11** may be configured to guide a respective tendon through a corresponding through-hole thereof. In the exemplary core skeleton layer **C,** tendon guide elements **11** may thereby define one or more tendon routing paths towards each end effector, e.g., towards each of the thumb sub-structure **30** and finger sub-structures **20.**

The core skeleton layer **C** may comprise at least one main layer section **12** having a first stiffness. The at least one main layer section **12** may be a physical section of the core skeleton layer **C.**

The core skeleton layer **C** may further comprise at least one reinforcement section **13** having at least a second stiffness greater than the first stiffness. The at least one reinforcement section **13** may be a physical section of the core skeleton layer **C** different from the at least one main layer section **12.** For example, at least one reinforcement section **13** may be implemented as a reinforcement rib and/or strut. Furthermore, at least one reinforcement section **13** may be provided proximal to each tendon routing path. For example, a reinforcement section **13** may be provided at least partially along each tendon routing path. Alternatively or additionally, each tendon guide element **11** may be mounted on and/or adjacent to at least one reinforcement section **13.**

The core skeleton layer **C** may further comprise at least one flexion section **14** having at least a third stiffness smaller than the first stiffness. The at least one flexion section **14** may be a physical section of the core skeleton layer **C** different from the at least one main layer section **12** and/or the at least one reinforcement section **13.** For example, the at least one flexion section **14** may comprise one or more through-holes through the core skeleton layer **C.**

The support structure **1** may further comprise one or more finger sub-structures **20,** wherein each finger sub-structure **20** may be configured to be worn on a corresponding finger **F** of the user, such as an index finger, a middle finger, a ring finger, and/or a little finger. Furthermore, the finger sub-structures **20** may be, preferentially releasably, connectable with and/or fixable to the hand body sub-structure **10.**

Each finger sub-structure **20** may further comprise at least one tendon guide element **21** configured to guide at least one tendon along the respective finger sub-structure **20.** Each tendon guide element **21** may comprise a corresponding through-hole (not shown for illustrative purposes), wherein the through-holes of the tendon guide elements **21** of a finger sub-structure **20** define a corresponding tendon routing path.

The support structure **1** may further comprise a thumb sub-structure **30,** wherein the thumb sub-structure **30** may be configured to be worn on a thumb **T** of the user. Furthermore, the thumb sub-structure **30** may be, preferentially releasably, connectable with and/or fixable to the hand body sub-structure **10.**

The thumb sub-structure **30** may further comprise at least one tendon guide element **31** configured to guide at least one tendon along the thumb sub-structure **30.** Each tendon guide element **31** may comprise a corresponding through-hole **32,** wherein the through-holes **32** of the tendon guide elements **31** of the thumb sub-structure **30** define a corresponding tendon routing path.

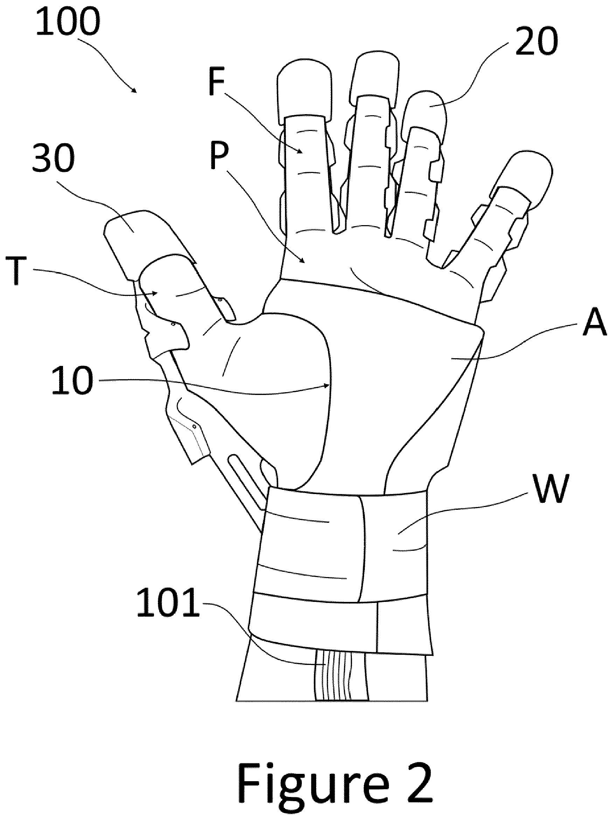

**Figure 2** shows a schematic view of a user wearable orthosis **100** comprising an exemplary support structure **1** according to Figure 1. In particular, the user wearable orthosis **100** is an exemplary tendon-driven glove orthosis **100** configured to be worn on a hand of a user.

It is noted that, for the sake of brevity, features already described in relation to Figure 1 will not be described again. Instead, corresponding reference is made to Figure 1, as described above.

In particular, it is noted that the wearable orthosis **100,** as shown in Figure 2, comprises the support structure **1,** as shown in Figure 1, and differs therefrom for example in the provision of one or more additional layers **A.**

Specifically, the support structure **1** and/or the hand body sub-structure **10** further comprises at least one additional layer **A** at least partially covering the core skeleton layer **C.** The at least one additional layer **A** may be arrangeable and/or fixable adjacent to the core skeleton layer **C.**

The additional layer **A** may be configured to be wrappable around the hand of the user. In particular, although not explicitly shown in Figure 2 for illustrative purposes, the additional layer **A** may comprise at least one first additional locking section provided on and/or in the additional layer **A** and at least one second additional locking section provided on and/or in the additional layer **A.** The at least one first additional locking section may be configured to be engageable with the at least one second additional locking section to mount the hand body sub-structure **10** and/or the additional layer **A** on the user.

In addition, further locking elements may be provided, wherein the locking elements may be configured to hold and/or fix at least the support structure **1,** the core skeleton layer C, and/or the at least one additional layer **A** on the hand of the user. For example, the locking elements may comprise a wrist belt **W,** wherein the wrist belt **W** may be configured to be wound around a wrist and/or lower arm of the user to hold and/or fix at least the support structure **1,** the core skeleton layer **C,** and/or the at least one additional layer **A** on the hand of the user.

In particular, the at least one additional layer **A** may comprise at least one outer additional layer **A.** The at least one outer additional layer **A** may be arrangeable at least partially on and/or fixable to an outer side of the core skeleton layer **C.** In particular, the outer side of the core skeleton layer **C** may be a side of the core skeleton layer **C** facing away from the user and/or the hand. In other words, the at least one outer additional layer **A** may be at least partially arrangeable such that the core skeleton layer **C** is arrangeable and/or positionable between the hand of the user and the at least one outer additional layer **A.**

In Figure 2, an outer additional layer **A** is shown as an outer fabric layer. The outer fabric layer may, for example, be configured to improve user comfort and safety. The outer fabric layer may be composed of different materials, such as for example a fleece-like fabric and a leather-type material. However, the outer fabric layer is not restricted to such a material composition, and other compositions may be possible, such as for example dependent on user requirements. The choice of materials of the outer fabric layer may serve to improve, for example, a comfort and/or aesthetic of the user wearable orthosis **100.**

In particular, functional elements may be provided on the at least one additional layer **A.** For example, one or more protective elements, such as one or more protective patches, and/or optical elements, such as one or more reflective elements, may be provided on the at least one additional layer **A.**

Furthermore, the at least one additional layer **A** may also comprise at least one inner additional layer **A.** However, due to illustrative purposes, such an at least one inner additional layer **A** is not shown in Figure 3.

Furthermore, the user wearable orthosis **100** may further comprise the at least one tendon. It is noted that, for illustrative purposes, no full-length tendon is shown. Instead, as an illustrative placeholder, multiple tendon sheaths **101** and/or Bowden cables are shown extending from the user wearable orthosis **100** in the direction (e.g., along the lower arm of the user) towards an actuator.

**Figure 3** shows a further exemplary core skeleton layer **C.** A support structure **1** of a user wearable, tendon-driven glove orthosis **100** may comprise the core skeleton layer **C.** Furthermore, for illustrative purposes, the at least one additional layer **A** is not shown in Figure 3.

The core skeleton layer **C** is configured to be worn on a hand of the user, wherein the core skeleton layer **C** may be configured to be wrapped around a palm **P** and a back of the hand of the user to mount at least the core skeleton layer **C** on the user.

The exemplary support structure **1** may further comprise a first locking section and a second locking section engageable with the first locking section, as described above. In the exemplary core skeleton layer **C** shown in Figure 3, no first and second core locking sections, as described above, are provided. Instead, at least one first and second additional locking section may be provided on and/or in one or more of the at least one additional layers (not shown), as described above.

Figure 3 shows the core skeleton layer **C** in a state, in which the core skeleton layer **C** is not mounted on the user, such as just after its manufacture (e.g., via 3D printing thereof). Specifically, the support structure **1** and/or the skeleton layer **C** may be configured to be substantially planar when not worn by the user. This allows, for example, a very efficient, rapid, and simple manufacture of the core skeleton layer **C,** such as by additive manufacturing.

The exemplary core skeleton layer **C** may be formed of at least one metal (e.g., low carbon steel), at least one elastomer, at least one polymer (e.g., silicone, thermoplastic urethane (TPU), thermoplastic elastomer (TPE), and/or PA-6 (polyamide 6)), and/or at least one non-textile material.

The core skeleton layer **C** may further comprise one or more reinforcement sections **13** and one or more flexion section **14,** as already described for example in relation to Figure 1. A duplicate description thereof is therefore omitted, and instead reference is made to the respective descriptions above.

The core skeleton layer **C** may be configured to guide at least one tendon between an actuation unit and at least one end effector mounted on the hand by defining at least one tendon routing path along the core skeleton layer **C,** wherein the at least one tendon may be moveable along the at least one tendon routing path while being restricted by the core skeleton layer **C** in movement lateral to said at least one tendon routing path.

The core skeleton layer **C** may further comprise at least one tendon guide element **11.** Each tendon guide element **11** may be configured to guide at least one tendon along the core skeleton layer **C,** wherein the at least one tendon may be at least partially guided within a central cavity or opening of the respective tendon guide element **11.** Each tendon guide element **11** may have one central cavity or opening to guide a tendon therein or may have a plurality of central cavities or openings to guide a plurality of tendons therein. One or more of the tendon guide elements **11** may define a corresponding tendon routing path for a respective tendon. Furthermore, the core skeleton layer **C** may further comprise at least one tendon groove **15.** Each tendon groove **15** may extend at least partially along a tendon routing path, and may further be configured to accommodate a respective tendon at least partially along its corresponding tendon routing path along the core skeleton layer **C.**

In the shown exemplary core skeleton layer **C,** five tendon routing paths are defined. Specifically, one tendon routing path is defined for each finger **F** and/or thumb **T** of the user. It is noted that the present disclosure is not limited to five tendon routing paths. Instead, the number of tendon routing paths may be determined on the basis of the number of required and/or desired tendons. Therefore, more or less than five tendon routing paths may be implemented.

The core skeleton layer **C** may further comprise one or more hardware mounting elements **16, 17A, 17B.** The one or more hardware mounting elements **16, 17A, 17B** may be configured such that one or more external hardware may be mountable to the core skeleton layer **C.** Examples of external hardware include, but are not limited to, control units and/or sensors.

The one or more hardware mounting elements **16, 17A, 17B** may be at least partially differently configured to one another, wherein a particular configuration of a hardware mounting element **16, 17A, 17B** may be chosen and/or implemented based on the specific requirements of the respective external hardware.

For example, a hardware mounting element **16** may be implemented as a mounting frame. The mounting frame may comprise a central cavity to at least partially accommodate the respective external hardware, such as a control unit. An edge around the central cavity may be reinforced and/or thickened to allow fixing of the respective external hardware thereto. The edge may for example be formed from the same material as the core skeleton layer **C.**

For example, the hardware mounting element **17A** may be implemented as a mounting hardpoint. The mounting hardpoint may be configured to fix an external hardware to the core skeleton layer **C,** wherein the mounting hardpoint may be formed from a material different to a material of the core skeleton layer **C.** In particular, a material of the mounting hardpoint may have a stiffness greater than a stiffness of the core skeleton layer **C.** The mounting hardpoint may for example comprise one or more latches and/or loops and/or hooks.

For example, the hardware mounting element **17B** may be configured to have an at least partially loop-like and/or tubular-like shape, wherein the hardware mounting element **17B** may be configured to fix and/or guide a respective external hardware, such as a power supply cable and/or data cable, to and/or along the core skeleton layer **C.** The hardware mounting element **17B** may be similarly configured to the at least one tendon guide element **11,** although it may differ therefrom in at least its dimensions and/or scale.

Alternatively or additionally to the provision of hardware mounting elements, such as shown hardware mounting elements **16, 17A, 17B,** hardware may also be embedded at least partially, preferably fully, into the core skeleton layer **C.** For example, it may be possible to embed one or more power and/or data transmission elements (not shown), such as cabling, into the core skeleton layer **C.** For example, the one or more at least partially embedded power and/or data transmission elements may comprise at least one conductive layer, e.g., a conductive polymer layer, in the core skeleton layer **C.** Such at least one conductive layer may, for example, be formed during the manufacture of the core skeleton layer **C,** such as during a 3D printing thereof.

At least the tendon guide elements **11** and one or more of the hardware mounting elements **16, 17A, 17B** may be integrally formed with the core skeleton layer **C.** For example, the tendon guide elements **11** and one or more of the hardware mounting elements **16, 17A, 17B** may be 3D printed during a 3D printing of the core skeleton layer **C.** Alternatively or additionally, at least one tendon guide element **11** and one or more of the hardware mounting elements **16, 17A, 17B** may be fixable to and/or mountable on the core skeleton layer **C.** For example, at least one tendon guide element **11** and one or more of the hardware mounting elements **16, 17A, 17B** may be 3D printed, using a different material to a material of the core skeleton layer **C,** onto the core skeleton layer **C** after its manufacture.

The core skeleton layer **C** may further comprise at least one mounting section **18,** wherein the at least one end effector may be fixable, preferentially releasably fixable, to the at least one mounting section **18.** For example, the core skeleton layer **C** may comprise a mounting section **18** configured such that a finger sub-structure **20** and/or a thumb sub-structure **30** may be fixable to the mounting section **18.** In particular, the at least one mounting section **18** may be at least partially engageable with the finger sub-structure **20** and/or a thumb sub-structure **30.** The at least one mounting section **18** may be formed form a different material than a material of the core skeleton layer **C,** preferentially from a material having a greater stiffness than the material of the core skeleton layer **C.**

The at least one mounting section **18** may comprise one or more engaging elements, such as one or more pegs as shown in Figure 3. The one or more engaging elements may be configured to be engageable with one or more corresponding engaging elements, e.g., such as one or more peg-receiving recesses, of the at least one end effector to fix the at least one end effector to the at least one mounting section **18.** Such an exemplary configuration of the at least one mounting section **18** may allow a reliable transfer of forces and/or a high resistance of connection quality and safety to degradation over time.

However, the at least one mounting section **18** is not limited to the exemplary embodiment shown in Figure 3, and may comprise, alternatively or additionally, different engaging elements. Further non-limiting examples of possible engaging elements may include one or more hardware mounting elements, such as exemplary hardware mounting elements **16, 17A, 17B,** and/or one or more clamping elements. For example, one or more exemplary hardware mounting elements **17A** may be provided such that one or more finger sub-structures **20** may be fixable thereto.

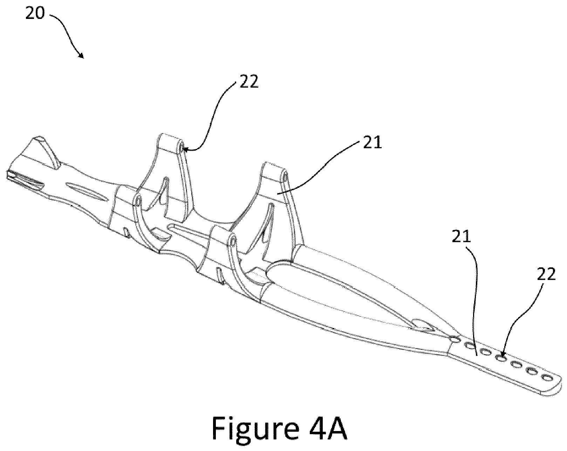

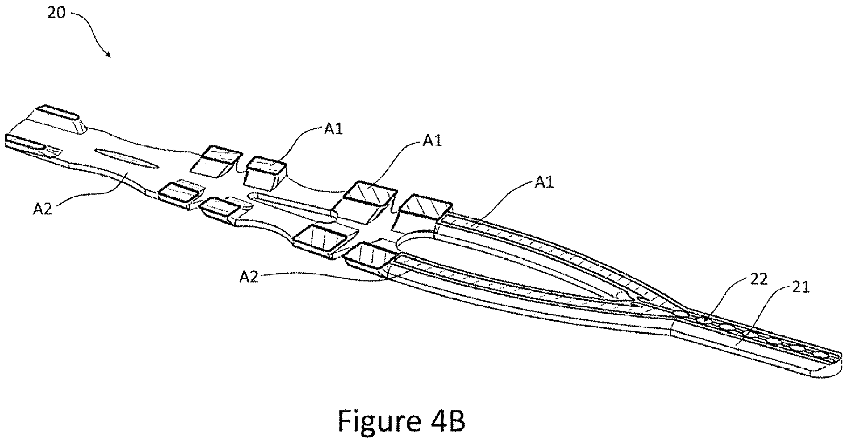

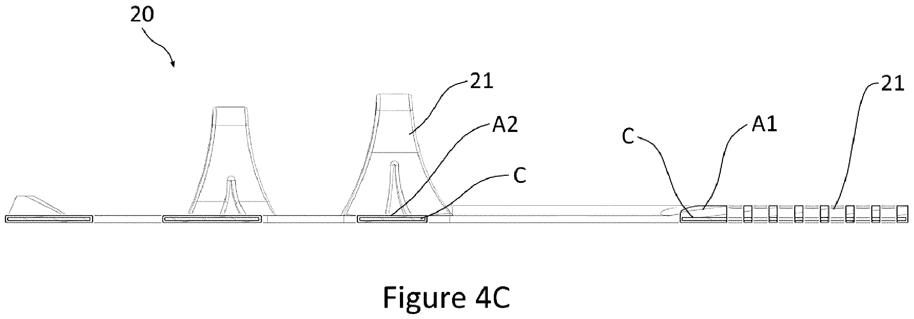

**Figure 4A** shows a schematic view of an exemplary support structure **1** having at least a finger sub-structure **20.** **Figure 4B** shows a horizontal cross-sectional view of the exemplary support structure **1** according to Figure 4A. **Figure 4C** shows a vertical cross-sectional view of the exemplary support structure **1** according to Figure 4A.

In particular, the support structure **1** comprises the finger sub-structure **20,** as shown in Figure 4A. The finger sub-structure **20** may be wearable on at least one finger **F** of the user. Furthermore, the finger sub-structure **20** may be configured to be releasably connectable to and/or fixable to a hand body sub-structure **10.**

Furthermore, the finger sub-structure **20** may comprise at least one, preferentially five, tendon guide element **21** configured to guide at least one tendon along the respective finger sub-structure **20.** Each tendon guide element **21** may comprise a corresponding through-hole **22,** wherein the through-holes **22** of the tendon guide elements **21** of the finger sub-structure **20** define a corresponding tendon routing path. Furthermore, a frontal tendon guide element **21** may be configured to be bent over the finger **F** of the user in the use state. The frontal tendon guide element **21** may comprise multiple through-holes **22** to allow the finger sub-structure **20** to be adaptable to different finger lengths of the finger **F.** Furthermore, the through-holes **22** of the frontal tendon guide element **21** may be substantially perpendicular to through-holes **22** of the other tendon guide elements **21.**

The support structure **1** and/or the finger sub-structure **20** further comprise a core skeleton layer **C** configured to guide at least one tendon along the core skeleton layer **C.** The core skeleton layer **C** may have a core stiffness. Furthermore, the exemplary core skeleton layer **C** shown in Figures 4A to 4C may be a core skeleton sub-layer of the finger sub-structure **20.** The core skeleton layer **C** may be formed of at least one metal such as a low-carbon stell sheet or at least one polymer such as PA-6.

The support structure **1** and/or the finger sub-structure **20** further comprise two additional layers **A1, A2** at least partially covering the core skeleton layer **C.** In particular, the two additional layers **A1, A2** may be fixed adjacent to the core skeleton layer **C.** Furthermore, the two additional layers **A1, A2** may be additional sub-layers of the finger sub-structure **20.** Each of the two additional layers **A1, A2** has a stiffness lower than a stiffness of the core skeleton layer **C.**

The two additional layers **A1, A2** comprise a first additional layer **A1.** In particular, the first additional layer **A1** may be a first surrounding additional layer **A1.** The first surrounding additional layer **A1** may be directly fixed to the core skeleton layer **C,** and may be configured to enclose the core skeleton layer **C** at least partially. In other words, the core skeleton layer **C** may be at least partially embedded in the first surrounding additional layer **A1.** The first additional layer **A1** may be formed from a low-density TPU/TPE foam material. The first additional layer A1 may be formed from a material having a low Shore hardness, such as having a Shore hardness in the range of approx. 20A to 80A, for example approx. 30A or approx. 65A.

The two additional layers **A1, A2** comprise a second additional layer **A2.** In particular, the second additional layer **A2** may be a second surrounding additional layer **A2.** The second surrounding additional layer **A2** may be directly fixed to the core skeleton layer **C** and the first additional layer **A1,** and may be configured to enclose the core skeleton layer **C** and the first additional layer **A1** at least partially. In other words, the core skeleton layer **C** and the first additional layer **A1** may be at least partially embedded in the second surrounding additional layer **A2.** The second additional layer **A2** may be formed from a low-density TPU/TPE foam material, preferentially the same material as the first additional layer **A1.**

The core skeleton layer **C,** the first additional layer **A1,** and the second additional layer **A2** may be formed by 3D printing. In particular, the skeleton layer **C,** the first additional layer **A1,** and the second additional layer **A2** may be substantially concurrently formed during the 3D printing.

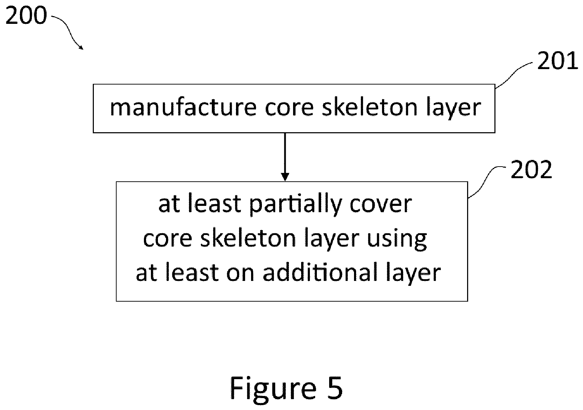

**Figure 5** shows an exemplary flow chart of a method **200** of manufacturing a user wearable orthosis **100.** The user wearable orthosis **100** may comprise a support structure **1** configured to be worn on a hand of a user.

In a first step **201,** a core skeleton layer **C** of the support structure **1** is manufactured, wherein the core skeleton layer **C** is configured to guide at least one tendon between an actuation unit and at least one end effector mounted on the hand.

In a second step **202,** the core skeleton layer **C** is at least partially covered with at least one additional layer **A.** The at least one additional layer **A** may have a stiffness lower than a stiffness of the core skeleton layer **C.**

The invention is not to be limited by any of the herein described and/or shown exemplary embodiments. Instead, the invention is defined by the independent claims, wherein preferred embodiments form the subject of the dependent claims.

### List of Reference Numerals

- **P**: Palm
- **F**: Finger
- **T**: Thumb
- **W**: Wrist belt
- **C**: Core skeleton layer
- **A, A1, A2**: Additional layer
- **100**: User wearable orthosis
- **101**: Tendon sheath
- **1**: Support structure
- **10**: Hand body sub-structure
- **11, 21, 31**: Tendon guide element
- **12**: Main layer section
- **13**: Reinforcement section
- **14**: Flexion section
- **15**: Tendon groove
- **16, 17A, 17B**: Hardware mounting element
- **18**: Mounting section
- **20**: Finger sub-structure
- **22, 32**: Through-hole
- **30**: Thumb sub-structure
- **200**: Method
- **201, 202**: steps

## Claims

1. A user wearable orthosis (100) comprising a support structure (1) configured to be worn on a hand of a user, wherein the support structure (1) comprises:
a core skeleton layer (C) configured to guide at least one tendon between an actuation unit and at least one end effector mounted on the hand;
at least one additional layer (A, A1, A2) at least partially covering the core skeleton layer (C),
wherein the at least one additional layer (A, A1, A2) has a stiffness lower than a stiffness of the core skeleton layer (C).

2. The user wearable orthosis (100) according to claim 1, wherein the support structure (1) is configured to be worn substantially on a palm (P) of the hand and a back of the hand.

3. The user wearable orthosis (100) according to one of the preceding claims, wherein the support structure (1) comprises a first locking section and a second locking section engageable with the first locking section, and
wherein the support structure (1) is configured to be wrapped substantially around the hand of the user to bring the first locking section and the second locking section into engagement with each other to mount the support structure (1) on the user.

4. The user wearable orthosis (100) according to one of the preceding claims, wherein the core skeleton layer (C) is formed of at least one metal, at least one elastomer, at least one polymer, and/or at least one non-textile material; and/or
wherein the at least one additional layer (A, A1, A2) is formed of at least one elastomer, at least one polymer, at least one leather, and/or at least one textile material.

5. The user wearable orthosis (100) according to one of the preceding claims, wherein the at least one additional layer (A, A1, A2) comprises:
at least one inner additional layer, wherein the at least one inner additional layer is arrangeable at least partially on an inner first side of the core skeleton layer (C), and, optionally,
wherein the at least one inner additional layer is detachable and/or removable from the core skeleton layer (C).

6. The user wearable orthosis (100) according to one of the preceding claims, wherein the at least one additional layer (A, A1, A2) comprises:
at least one outer additional layer, wherein the at least one outer additional layer is arrangeable at least partially on an outer second side of the core skeleton layer (C), and, optionally,
wherein the at least one outer additional layer is detachable and/or removable from the core skeleton layer (C).

7. The user wearable orthosis (100) according to one of the preceding claims, wherein the core skeleton layer (C) is manufactured using molding or additive manufacturing.

8. The user wearable orthosis (100) according to one of the preceding claims, wherein the core skeleton layer (C) further comprises at least one tendon guide element (11, 21, 31), and
wherein the at least one tendon guide element (11, 21, 31) is configured to guide the at least one tendon along the core skeleton layer (C).

9. The user wearable orthosis (100) according to one of the preceding claims, wherein the core skeleton layer (C) comprises at least one main layer section (12) having a first stiffness,
wherein the core skeleton layer (C) further comprises one or more of:
at least one reinforcement section (13) having at least a second stiffness greater than the first stiffness; and
at least one flexion section (14) having at least a third stiffness smaller than the first stiffness.

10. The user wearable orthosis (100) according to one of the preceding claims, wherein the user wearable orthosis (100) further comprises the at least one end effector, and, optionally,
wherein the at least one end effector is configured to be worn on one or more of a thumb (T), an index finger, a middle finger, a ring finger, and a little finger of the hand of the user.

11. A method (200) of manufacturing a user wearable orthosis (100) comprising a support structure (1) configured to be worn on a hand of a user, comprising:
manufacturing a core skeleton layer (C) of the support structure (1) configured to guide at least one tendon between an actuation unit and at least one end effector mounted on the hand;
at least partially covering the core skeleton layer (C) with at least one additional layer (A, A1, A2),
wherein the at least one additional layer (A, A1, A2) has a stiffness lower than a stiffness of the core skeleton layer (C).

12. The method (200) according to claim 11, further comprising:
wrapping the support structure (1) substantially around the hand to bring a first locking section of the support structure (1) into engagement with a second locking section of the support structure (1) to mount the support structure (1) on the user.

13. The method (200) according to one of claims 11 to 12, wherein manufacturing the core skeleton layer (C) comprises manufacturing the core skeleton layer (C) using molding and/or additive manufacturing.

14. The method (200) according to one of claims 11 to 13, wherein manufacturing the core skeleton layer (C) further comprises providing at least one tendon guide element (11, 21, 31) on and/or in the core skeleton layer (C),
wherein the at least one tendon guide element (11, 21, 31) is configured to guide the at least one tendon along the core skeleton layer (C).

15. The method (200) according to one of claims 11 to 14, wherein manufacturing the core skeleton layer (C) further comprises forming at least one main layer section (12) having a first stiffness, and
wherein manufacturing the core skeleton layer (C) further comprises one or more of:
forming at least one reinforcement section (13) having at least a second stiffness greater than the first stiffness; and
forming at least one flexion section (14) having at least a third stiffness smaller than the first stiffness.
